# EUROPEAN PATENT SPECIFICATION

(11) **EP 1 850 130 B1**
(45) Date of publication and mention of the grant of the patent: **02.11.2011**
(21) Application number: 06702618.7
(22) Date of filing: 13.01.2006
(51) Int. Cl.: G01N 33/50, C12N 15/09, C12Q 1/68, G01N 33/15, G01N 33/53

(54) **GENE MARKERS AND UTILZATION OF THE SAME**
GENMARKER UND DESSEN VERWENDUNG
MARQUES DE GENE ET LEUR UTILISATION

(30) Priority: 13.01.2005 JP 2005006728
(43) Date of publication of application: 31.10.2007
(73) Proprietor: Keio University, Tokyo 108-8345 (JP)
(72) Inventor: TANIGAWARA, Yusuke, c/o Keio University School of Medicine, Tokyo, 1608582 (JP); NOMOTO, Maiko, c/o Keio University School of Medicine, Tokyo, 1608582 (JP); MIHARA, Kiyoshi, c/o Keio University School of Medicine, Tokyo, 1608582 (JP); IKETANI, Osamu, c/o Keio University School of Medicine, Tokyo, 1608582 (JP); TANABE, Minoru, c/o Keio University School of Medicine, Tokyo, 1608582 (JP); HOSHINO, Ken, c/o Keio University School of Medicine, Tokyo, 1608582 (JP); SHIMAZU, Motohide, c/o Keio University School of Medicine, Tokyo, 1608582 (JP); MORIKAWA, Yasuhide, c/o Keio University School of Medicine, Tokyo, 1608582 (JP)
(74) Representative: Jones, Helen M.M.
(86) International application number: PCT/JP2006/300336
(87) International publication number: WO 2006/075682

(56) References cited:
- JP-A- 2001 517 459
- PAN T L ET AL: "Ceruloplasmin, a novel candidate as a diagnostic marker for liver function after liver transplantation." TRANSPLANTATION PROCEEDINGS NOV 2000, vol. 32, no. 7, November 2000 (2000-11), page 2198, XP002479179 ISSN: 0041-1345
- BOUDINOT ET AL.: 'Vesicular stomatitis virus and pseudorabies virus induce a vig1/cig5 homologue in mouse dendritic cells via different pathways' J. GEN. VIROL. vol. 81, November 2000, pages 2675 - 2682, XP003020018
- CHIN K.C. ET AL.: 'Cresswell P. Viperin (cig5), an IFN-inducible antiviral protein directly induced by human cytomegalovirus' PROC. NATL. ACAD. SCI. USA vol. 98, no. 26, 18 December 2001, pages 15125 - 15130, XP003020019
- GREWAL ET AL.: 'Best5: a novel interferon-inducible gene expressed during bone formation' FASEB J. vol. 14, no. 3, March 2000, pages 523 - 531, XP002208786

### SEQUENCE LISTING

<110> KEIO UNIVERSITY
<120> GENE MARKER AND UTILIZATION OF THE SAME
<130> HMJ04832EP
<140> EP 06702618.7 <141> 2006-01-13
<150> JP 2005/6728 <151> 2005-01-13
<160> 9
<170> Patentin version 3.1
<210> 1
   <211> 18
   <212> DNA
   <213> Artificial Sequence
<220>
   <223> Probe
<400> 1
   ccatcacacc ctggtgcc
   18
<210> 2
   <211> 17
   <212> DNA
   <213> Artificial sequence
<220>
   <223> Primer
<400> 2
   gccgtcttcc cctccat
   17
<210> 3
   <211> 22
   <212> DNA
   <213> Artificial Sequence
<220>
   <223> Primer <400> 3
<210> 4
   <211> 23
   <212> DNA
   <213> Artificial sequence
<220>
   <223> Primer
<400> 4
   ggtgcctgaa tctaaccaga aga
   23
<210> 5
   <211> 20
   <212> DNA
   <213> Artificial sequence
<220>
   <223> Primer
<400> 5
   acttggaagg gtccttccgt
   20
<210> 6
   <211> 19
   <212> DNA
   <213> Artificial sequence
<220>
   <223> Probe
<400> 6
   atgaatatat gcgctttct
   19
<210> 7
   <211> 21
   <212> DNA
   <213> Artificial Sequence
<220>
   <223> Probe
<400> 7
   caggcaccag ggcgtgatgg t
   21
<210> 8
   <211> 16
   <212> DNA
   <213> Artificial Sequence
<220>
   <223> Primer
<400> 8
   gccgccagct caccat
   16
<210> 9
   <211> 21
   <212> DNA
   <213> Artificial Sequence
<220>
   <223> Primer
<400> 9
   aggaatcctt ctgacccatg c
   21

## Description

### TECHNICAL FIELD

The present invention relates to gene markers and methods for diagnosing rejection, methods for judging the presence or absence of immunological tolerance, methods for evaluating the efficacy of immunosuppressive agents, methods for identifying immunosuppressive agents, and methods for screening for immunosuppressive agents or immunological tolerance-inducing agents.

### BACKGROUND ART

Organ transplantation is commonly performed as a therapeutic strategy for saving lives of patients with severe organ failure and for improving their QOL (quality of life). However, because of rejection, which is a serious complication, a transplanted organ is sometimes lost, so that the therapy turns out to be unsuccessful. For this reason, it is expected to develop methods for quickly diagnosing rejection, agents for controlling rejection (immunosuppressive agents), and the like with a view to taking early preventive measures against rejection.

As a method for diagnosing rejection after organ transplantation, tissue biopsies (needle biopsies) under local anesthesia are regarded as the most reliable. However, since this method is invasive and imposes a great burden upon patients, repeated treatment is impossible. Moreover, since performing a needle biopsy requires skilled technique and adequate equipment, it cannot be performed easily. Currently, in place of the needle biopsies, there have been used other methods such as blood tests (for example, methods for measuring a change in concentration of white blood cells (WBC), CRP (C-reactive protein), organopathy markers (AST (aspartate aminotransferase;GOT)or ALT(alanine aminotransferase;GPT)), bilirubin, gamma GTP (guanosine triphosphate), creatinine, or blood urea nitrogen (BUN) in blood), Doppler ultrasonography (observation of blood stream), etc. However, all these are only auxiliary methods; none of these alone can diagnose the presence or absence of rejection. Therefore, there is a demand for the development of biomarkers for diagnosing the presence or absence of rejection in place of needle biopsies.

Biomarkers for evaluating rejection of transplanted tissues previously identified include perforin, granzyme B, Fas ligand, etc. (refer to National Publication of International Patent Application No. 2001-517459).

After the transplantation, in order to suppress the above-described rejection, an immunosuppressive agent is administered to the patient who has undergone transplantation. Immunosuppressive agents currently in use include cyclosporine (refer to e.g., Japanese patent No. 3382656), FK506 (tacrolimus: refer to, e.g., U.S. Patent No. 4894366), etc. These immunosuppressive agents cause adverse side effects such as infection because of overdosing; therefore it is absolutely essential to precisely determine the usage (modes of administration) and dosage (doses and dosing intervals) of each immunosuppressive agent.

The doses of immunosuppressive agents are determined by monitoring whether the blood concentrations of the drugs are within the effective blood concentrations of the drugs (therapeutic drug monitoring: TDM). However, such effective blood concentrations are determined empirically, not based on scientific evidence, and therefore do not necessarily serve as indicators of the efficacy.

On the other hand, there are a small number of patients whose rejection is reduced to the level where they no longer need an immunosuppressive agent and who can stop taking the immunosuppressive agent. Tolerance induction like this is considered to occur partly because immunosuppressive agents have tolerance-inducing activity. Since those patients who have acquired immunological tolerance can stop taking an immunosuppressive agent, they can be released from the risk of infection and malignant tumors and adverse side effects such as organopathy associated with long-term administration of an immunosuppressive agent. Accordingly, in recent years, development of methods have been attempted for artificially inducing immunological tolerance by, for example, inhibiting the co-stimulatory molecule CD28 expressed in naive T cells by administering an antibody or a recombinant protein. Alternatively, there are other attempts to induce immunological tolerance by elaborating the mode of administration of an immunosuppressive agent or by administering a plurality of immunosuppressive agents (in which case, immunosuppressive agent(s) are serving as tolerance-inducting agents as well).

However, whether or not immunological tolerance has been acquired cannot be correctly determined, because there is no scientific indicator of immunological tolerance; disadvantageously, a discontinuation of administration of immunosuppressive agent (s) in judging the presence or absence of acquisition of immunological tolerance has always entailed the danger of causing rejection. Therefore, there is a demand for the development of methods that make it possible to judge the presence or absence of immunological tolerance in a quick, simple, and convenient manner.

### DISCLOSURE OF THE INVENTION

### PROBLEMS TO BE SOLVED BY THE INVENTION

The present invention has been made in consideration of the above-mentioned problem. The object of the present invention is to provide gene markers that enables diagnosis of rejection, evaluation of drug efficacy, and judgment of the presence or absence of immunological tolerance; methods that can be performed in a quick, simple, and convenient manner using the gene markers as indicators for diagnosing rejection, for judging the presence or absence of immunological tolerance, for evaluating the efficacy of an immunosuppressive agent, and for identifying an immunosuppressive agent; and methods for screening for an immunosuppressive agent or an immunological tolerance-inducing agent.

### MEANS FOR SOLVING THE PROBLEMS

To solve the above-mentioned problem, the inventors of the present application have identified genes (Bc1211, Best5, Igifbp, Gbp2, etc.) whose expression levels were increased because of rejection and whose expression levels were decreased because of administration of an immunosuppressive agent, by analyzing gene expression patterns in the peripheral blood of rejection model rats using microarrays at early stages of acute rejection and at time of administration of an immunosuppressive agent. These genes are found to be useful as early markers because their expression increase before a rejection is pathologically diagnosed. Thus, the present invention has been accomplished.

Namely, the gene marker according to the present invention serves as an indicator of rejection, and the gene marker is a gene-related substance related to the gene cig5.
The gene marker serves as an indicator of rejection due to transplantation of a tissue or an organ, such as a liver, etc.

Further, the gene marker according to the present invention serves as an indicator of immunological tolerance, and the gene marker is a gene-related substance related to the gene cig5. For example, the gene marker serves as an indicator of immunological tolerance to transplantation of a tissue or an organ, such as a liver, etc.

Further, the gene marker according to the present invention serves as an indicator of the efficacy of an immunosuppressive agent, and the gene marker is a gene-related substance related to the gene cig5. For example, the gene marker serves as an indicator of the efficacy of an immunosuppressive agent on transplantation of a tissue or an organ, such as a liver, etc.

A method for diagnosing rejection according to the present invention diagnoses rejection by measuring a change in the expression level of a gene marker in blood drawn from a vertebrate other than a human, and the gene marker to be used is a gene-related substance related to the gene cig5. For example, the method for diagnosing rejection according to the present invention can examine the presence or absence of rejection due to transplantation of a tissue or an organ, such as a liver, etc.

Further, the kit for diagnosing described herein includes a primer or an antibody for measuring a change in the expression level of a gene marker in blood, and the gene marker to be used is a gene-related substance related to the gene cig5. For example, the kit for diagnosing rejection can examine the presence or absence of rejection due to transplantation of a tissue or an organ, such as a liver, etc.

A method for judging the presence or absence of immunological tolerance according to the present invention judges the presence or absence of immunological tolerance by measuring a change in the expression level of a gene marker in blood drawn from a vertebrate other than a human into which a tissue or an organ has been transplanted. The gene marker to be used is a gene-related substance related to the gene cig5.

The aforementioned tissue or organ is, for example, a liver, etc.

Further, the method for judging the presence or absence of immunological tolerance according to the present invention includes the steps of: monitoring the expression level of a gene marker in blood drawn from a vertebrate into which a tissue or an organ has been transplanted while reducing the dose of an immunosuppressive agent administered to the vertebrate after the transplantation; and judging that the immunological tolerance has been acquired in the case that no significant change in the expression level is found while the dose is reduced and withdrawal from the immunosuppressive agent has been achieved. The gene marker to be used is, a gene-related substance related to the gene cig5. The aforementioned tissue or organ is, for example, a liver, etc.

A kit for judging the presence or absence of immunological tolerance includes a primer or an antibody for measuring a change in the expression level of a gene marker in blood, and the gene marker to be used is a gene-related substance related to a gene selected from the group consisting of Bc1211, cig5, I118bp, and Gbp2. For example, there is described a kit for judging the presence or absence of immunological tolerance to transplantation of a tissue or an organ, such as a liver, etc.

The method for evaluating the efficacy of an immunosuppressive agent according to the present invention evaluates the efficacy of an immunosuppressive agent by measuring a change in the expression level of a gene marker in blood associated with a use of the immunosuppressive agent, and the gene marker to be used is a gene-related substance related to the gene cig5. For example, the present invention may be a method for evaluating the efficacy of an immunosuppressive agent on transplantation of a tissue or an organ, such as a liver, etc.

The method for identifying an immunosuppressive agent for a vertebrate requiring administration of an immunosuppressive agent according to the present invention includes the steps of: drawing blood from a plurality of individuals of the vertebrate family before and after administering an equal amount of different immunosuppressive agents; measuring the expression level of a gene marker in the blood; comparing the expression levels of the gene marker in the blood drawn from the individual between before and after administering the immunosuppressive agent; and identifying the immunosuppressive agent which has decreased the expression level of the gene marker most markedly by the administration of the immunosuppressive agent. The gene marker to be used is a gene-related substance related to the gene cig5. For example, the present invention may be a method for identifying an immunosuppressive agent effective for transplantation of a tissue or an organ, such as a liver, etc.

The method for selecting an immunosuppressive agent according to the present invention is to select the most effective immunosuppressive agent for an individual requiring an immunosuppressive agent among a plurality of the immunosuppressive agents by using calibration curves constructed for each of the plurality of immunosuppressive agents which represent correlations between the amount used or the blood concentration of the immunosuppressive agents and the expression level of a gene marker in the vertebrate individual requiring administration of an immunosuppressive agent. The gene marker to be used is a gene-related substance related to the gene cig5.

The aforementioned individual is preferably the one that has undergone transplantation of a tissue or an organ, such as a liver, etc.

The method for determining the dose of an immunosuppressive agent according to the present invention includes the steps of: drawing blood from a vertebrate with a disease or rejection which requires administration of the immunosuppressive agent; measuring the expression level of a gene marker in the blood; and determining the dose of the immunosuppressive agent in the vertebrate which is necessary to decrease the expression level of the gene marker in the blood of the vertebrate to a predetermined expression level by using a previously constructed calibration curve which represents a correlation between the amount used or the blood concentration of the immunosuppressive agent and the expression level of the gene marker. The gene marker to be used is a gene-related substance related to the gene cig5. The aforementioned individual is preferably the one that has undergone transplantation of a tissue or an organ, such as a liver, etc.

A kit for evaluating the efficacy of an immunosuppressive agent described herein includes a primer or an antibody for measuring a change in the expression level of a gene marker in blood, the change being associated with a use of the immunosuppressive agent, and the gene marker to be used is a gene-related substance related to the gene cig5. The aforementioned immunosuppressive agent may be the one that is used after transplantation of a tissue or an organ, such as a liver, etc.

Further, the screening method for either screening for an immunosuppressive agent or screening for an immunological tolerance-inducing agent according to the present invention includes the steps of: administering a test substance to a vertebrate in which a tissue or organ transplant causes immunological rejection; drawing blood from the vertebrate; and monitoring the expression level of a gene marker in the blood. The gene marker to be used is a gene-related substance related to a gene cig5. The aforementioned tissue or the organ is, for example, a liver, etc.

The method for suppressing rejection according to the present invention includes the steps of measuring the expression level of a gene marker while monitoring the blood concentration of an immunosuppressive agent, and administering the immunosuppressive agent or an immunological tolerance-inducing agent until the expression level is reached to a predetermined value. The method makes it possible to effectively suppress rejection caused by tissue or organ transplantation.

Further, the method for improving or treating an immunological disease according to the present invention includes the steps of measuring the expression level of a gene marker while monitoring the blood concentration of an immunosuppressive agent, and administering the immunosuppressive agent or an immunological tolerance-inducing agent until the expression level is reached to a predetermined value. The method makes it possible to effectively improve or treat immunological diseases such as autoimmune diseases, allergic diseases, atopic diseases, rheumatic diseases, pollinosis, etc.

It should be noted that the term "gene marker" as used herein refers to a gene-related substance serving as an indicator for evaluating the state or action of a particular object having a correlation with the expression level of a particular gene. The gene marker is the cig5 gene itself, an mRNA as a transcript, a peptide as a translation product, a protein as an end product of gene expression, etc. The "expression level of a gene marker" as used herein refers to, when a gene marker other than a gene is used, the transcription level (when the marker is a transcript etc.) of a gene from which the gene marker is derived, or the amount of expression (when the marker is a polypeptide or a protein, etc.) at the expression level. The aforementioned gene is not limited in terms of the species from which it is derived, and all of its homologs and orthologs are also encompassed. A "vertebrate" as used herein refers to a human and a vertebrate other than a human, such as a mouse, a rat, etc.

### BRIEF DESCRIPTION OF THE DRAWINGS

FIG. 1 shows the expression levels of the gene markers (Best5 and Bc1211) in the non-rejection group, immunosuppression group, and rejection group on 7POD in one Example.
FIG. 2 shows temporal changes in the expression level of the gene marker (Best5) in the non-rejection group, immunosuppression group, and rejection group in one Example.
FIG. 3 shows temporal changes in the expression level of the gene marker (Bc1211) in the non-rejection group, immunosuppression group, and rejection group in one Example.
FIG. 4 shows the results from an examination of the averages of the expression level of the gene (Best5) for each group obtained by picking up the highest values from the data by 3POD in one Examples.
FIG. 5 shows the results from an examination of the averages of the expression level of the gene (Bc1211) for each group obtained by picking up the highest values from the data by 3POD in one Example.
FIG. 6 shows the expression levels of the gene markers (Igifbp and Gbp2) in the non-rejection group, immunosuppression group, and rejection group on 5POD in one Example.
FIG. 7 shows changes in expression of the cig5 gene (upper figure) and liver function test values (lower figure) during acute rejection in a case of orthotopic liver transplantation in one Example according to the present invention.
FIG. 8 shows changes in expression of the BCL2L1 gene (upper figure) and liver function test values (lower figure) during acute rejection in a case of living liver transplantation in one Example.
FIG. 9 shows changes in expression (upper figure) and liver function test values (lower figure) of the IL18bp gene during acute rejection in a case of living liver transplantation in one Example.
FIG. 10 shows changes in expression of the GBP2 gene (upper figure) and liver function test values (lower figure) during acute rejection in a case of living liver transplantation in one Example.

### BEST MODE FOR CARRYING OUT THE INVENTION

Embodiments of the present invention accomplished based on the above-described findings are hereinafter described in detail by giving Examples. Unless otherwise explained, methods described in standard sets of protocols such as J. Sambrook and E. F. Fritsch & T. Maniatis (Ed.), "Molecular Cloning, a Laboratory Manual (3rd edition), Cold Spring Harbor Press and Cold Spring Harbor, New York (1989); and F. M. Ausubel, R. Brent, R. E. Kingston, D. D. Moore, J. G. Seidman, J. A. Smith, and K. Struhl (Ed.),"Current Protocols in Molecular Biology", John Wiley & Sons Ltd., and/or their modified/changed methods are used. When using commercial reagent kits and measuring apparatus, unless otherwise explained, protocols attached to them are used.

The object, characteristics, and advantages of the present invention as well as the idea thereof will be apparent to those skilled in the art from the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein wi thin the intent and scope of the present invention disclosed herein.

### Usefulness of gene markers ==

The gene marker according to the present invention is a gene-related substance which is related to the cig5 gene whose expression changes because of rejection or administration of an immunosuppressive agent. For example, when the gene marker to be used is a gene-related substance related to a gene selected from the group consisting of Bc1211, cig5, I118bp, and Gbp2, the expression levels of these gene markers are increased in blood before a rejection is pathologically diagnosed by liver biopsy, etc., and are decreased in blood by administration of an immunosuppressive agent. Therefore, the gene marker according to the present invention is useful as an early marker (indicator) of rejection, and is also useful as an indicator of immunological tolerance or as an indicator of the efficacy of an immunosuppressive agent. Also, the expression levels of the gene marker is inferred to change depending on the degree of rejection and the dose and blood concentration of an immunosuppressive agent. This suggests that the expression levels of the gene marker have a correlation with the degree of rejection as well as the dose and blood concentration of an immunosuppressive agent. It should be noted that the expression level of the gene marker may correlate inversely with rejection or an immunosuppressive agent.

Further, a plurality of the gene markers may be used for comprehensive diagnosis of rejection, as indicators of the efficacy of an immunosuppressive agent, or for judgment of the presence or absence of immunological tolerance. When a plurality of the gene markers are used in these ways, it is expected that more precise diagnosis of rejection, more strict evaluation of efficacy, and more precise judgment of the presence or absence of immunological tolerance are possible.

Moreover, by using a gene marker in blood, the blood can be used as a sample, thereby making it possible to periodically measure the expression level of the gene marker in a simple and convenient manner. This enables quick diagnosis of whether rejection occurs before rejection that is pathologically diagnosed is observed. This early diagnosis is extremely important in that it enables early preventive measures against rejection, thereby minimizing damage to the transplanted tissue or organ caused by rejection. In addition, drawing blood samples is less invasive and less technically demanding than needle biopsy, and thus can be performed in a simple and convenient manner. Likewise, use of blood as samples allows repeated evaluation of efficacy and diagnosis of immunological tolerance according to the present invention in a quick, simple, and convenient manner.

### == Quantification of gene markers ==

In embodiments where the gene marker is a gene or a transcript (mRNA) of a gene, the expression level of the gene marker can be measured (quantified) by measuring the amount of mRNA by, for example, the Northern blotting method, dot blotting method, quantitative reverse transcription-polymerase chain reaction (RT-PCR) method, etc. It is preferred to use the quantitative RT-PCR method in that the method enables measurement of the amount of mRNA by using a very small amount of RNA (isolated from leukocytes).

The primer to be used for the quantitative RT-PCR method is not particularly limited, as long as it can specifically detect the gene marker, but an oligonucleotide consisting of 12 to 26 nucleotides is preferred. The nucleotide sequence is determined based on the sequence information of the following genes of the vertebrate to be examined: Bc1211 (BCL 2-like 1), cig5 (also known as RSAD2 (radical S-adenosyl methionine domain containing 2), Best5 in rats); I118bp (interleukin-18-binding protein, Igifbp in rats (interferon gamma inducing factor binding protein)); and Gbp2 (Guanylate-binding protein 2) . Then, the primer containing the determined sequence can be prepared with, for example, a DNA synthesizer. To be specific, for example, when the animal is a human, the primer can be prepared based on the sequence information deposited as NM001191 and NM138578 (BCL2L1), NM080657 (cig5), NM005699 and NM173042-NM173044 (IL18BP), and NM004120 (GBP2). When the animal is a rat, the primer can be prepared based on the sequence information deposited as NM031535 (Bc1211), NM138881 (Best5), NM053374 (Igifbp), and NM133624 (Gbp2).

On the other hand, when the gene marker is a translation product (a polypeptide) or an end product (a protein) of the gene, the expression level of the gene marker can be measured by, for example, the Western blotting method, the ELISA method, etc., which uses a polyclonal antibody, a monoclonal antibody, etc., specific to the gene marker. However, the measurement method is not limited to these; many other techniques, such as radioimmunoassay (RIA), enzyme immunoassay (EIA), can be used. Hereinbelow, measurement of the expression level of a gene marker will be explained by an example of the ELISA method using a polyclonal antibody.

Blood drawn from a vertebrate is homogenized, and then ultrasonicated. After centrifugation, the supernatant is recovered to obtain a crude extract containing proteins. Then, by using an ELISA plate (e.g., 96-well plate) coated with a polyclonal antibody which specifically binds to the gene marker, the crude protein extract is placed in the wells of the plate for reaction with the gene marker, and, subsequently, with the primary antibody and then secondary antibody (e.g., an enzyme-labeled anti-immunoglobulin antibody). After the reactions, the ELISA plate is washed, color is developed with a substrate for the enzyme, the absorbance is measured with a microplate reader, and the expression level of the protein is quantified by using a previously constructed calibration curve. The expression level of a gene marker can be measured in this manner. The enzyme to be used to label the secondary antibody is exemplified by, but not limited to, horseradish peroxidase (HRP), alkaline phosphatase, etc.

### == Method for diagnosing rejection ==

When a gene marker as described above is used, rejection can be diagnosed from early stages by, for example, periodically measuring the expression level of the gene marker in blood drawn from a vertebrate into which a tissue or an organ has been transplanted, and comparing the expression level with an expression level of the gene marker in blood drawn before the tissue or organ transplantation. That is, as a result of measuring the expression levels of the gene marker, if the post-transplantation expression level of the marker has significantly changed compared with the pre-transplantation expression level, or the expression level of the gene marker is temporally changing significantly, it is judged that rejection has occurred or is likely to occur. For example, when the gene marker is s a gene-related substance related to a gene selected from the group consisting of Bc1211, cig5, I118bp, and Gbp2, if the expression level has increased significantly compared with the pre-transplantation expression level or is temporally increasing significantly, it is judged that rejection has occurred or is likely to occur. On the other hand, if the post-transplantation expression level of the marker has not changed significantly compared with the pre-transplantation expression level, it is judged that rejection has not occurred. A vertebrate to be diagnosed may be a human or a vertebrate other than a human, such as a mouse, a rat, or the like. == Method for evaluating the efficacy of an immunosuppressive agent ==

As described above, the expression level of the gene marker according to the present invention is useful not only as an indicator of rejection but also as an indicator in evaluating the efficacy of an immunosuppressive agent. If it becomes possible to evaluate the efficacy of an immunosuppressive agent by measuring the expression level of a gene marker, the mode of administration of an immunosuppressive agent for individual disease can be easily determined based on scientific evidence. For example, it becomes possible to select an immunosuppressive agent and/or mode of its administration effective in a small dose with minimal adverse side effects.

It should be noted that the diseases to be treated with immunosuppressive agents include immunological diseases such as autoimmune diseases, allergic diseases (such as allergic dermatitis, atopic diseases, pollinosis, etc.), and rheumatic diseases, besides rejection.

The immunosuppressive agent is not limited to any specific one as long as it is an agent, for example, an agent that suppresses abnormal immunological reactions, such as rejection in the living body after transplantation, or an agent that suppresses immunity in various diseases. Examples of the immunosuppressive agent include steroids (prednisolone, methylprednisolone, etc.), IL-2 antibodies, IL-2 secretion suppressors (rapamycin, etc.), CN suppressors such as cyclosporine or FK506 (also referred to as tacrolimus or Prograf), antimetabolite agents (mycophenolate mofetil, etc.), cyclophosphamide, OKT3 (also referred to as muromonab-CD3), antilymphocytic globulin (antithymocyte immunoglobulin), anti-CD4 antibodies, anti-TNF-a antibodies, azathioprine, mizoribine, sulfasalazine, 6-mercaptopurine (6-MP), methotrexate, cytoxazone, gusperimus hydrochloride, or combinations of these agents.

### == Determination of the mode of administration of an immunosuppressive agent ==

In a vertebrate individual that has received an immunosuppressive agent after suffering from the aforementioned disease or undergoing a transplantation surgery, by measuring the expression level of a gene marker in blood (or peripheral blood) of the vertebrate while monitoring the dose of the immunosuppressive agent or the blood concentration of the immunosuppressive agent and, administering the immunosuppressive agent until the expression level is reached to a predetermined value (e.g. until the difference from a normal level decreased to about 20%), it becomes possible to improve or treat the aforementioned disease or to suppress rejection using a minimal dose of the immunosuppressive agent. This enables effective prevention of side effects such as infection associated with overdosing of the immunosuppressive agent. It should be noted that for initial dose of an immunosuppressive agent, an exemplary amount is the minimum dose of the immunosuppressive agent which is determined based on a calibration curve described below.

### == Construction and application of a calibration curve ==

Constructing a calibration curve representing a correlation between the expression level of a gene marker and the dose or the blood concentration of an immunosuppressive agent in a vertebrate individual which has received the immunosuppressive agent makes it possible to evaluate efficacy of the immunosuppressive agent from various aspects.

For example, it becomes possible to select the most useful immunosuppressive agent for each of the various vertebrates with immunological disease or rejection, by selecting an immunosuppressive agent which brings the expression level of the gene marker to the normal level in a smallest quantity, or by selecting an immunosuppressive agent by which the largest difference occurs between the dose which causes a side effect and the dose which brings the expression level of the gene marker to the normal level, based on calibration curves constructed for a plurality of immunosuppressive agents.

In addition, by using calibration curves which have been constructed as described above for a plurality of modes of administration, the most effective administration mode can be selected. The modes of administration of an immunosuppressive agent include, for example, an injection to an affected area, subcutaneous, intramuscular, intraperitoneal, or intravenous injections, oral administration, parenteral administrations such as applying and pasting, etc.

Also, for vertebrates with immunological diseases such as autoimmune diseases, allergic diseases, atopic disease, rheumatic disease, and pollinosis, or with rejection, which requires administration of an immunosuppressive agent, it becomes possible to establish an optimal dose of the immunosuppressive agent by using the calibration curve as described above. For example, in a particular human or animal patient, by calculating the difference between the expression level of the gene marker before administration of the immunosuppressive agent and a normal expression level of the gene marker, it is possible to determine the dose of an immunosuppressive agent which can decrease the expression level of a gene marker only by a certain percentage.

### == Method for judging the presence or absence of immunological tolerance ==

According to the method for judging the presence or absence of immunological tolerance according to the present invention, it is possible to judge the presence or absence of immunological tolerance by measuring a change in the expression level of a gene marker in blood drawn from a vertebrate into which a tissue or an organ has been transplanted.

One example of the method for judging the presence or absence of immunological tolerance is the method including monitoring the expression level of a gene marker in blood drawn from a vertebrate into which a tissue or an organ has been transplanted, while reducing the dose of the immunosuppressive agent administered to the vertebrate. Namely, as a result of measuring the expression level of a gene marker, if the expression level of the gene marker shows the same change as that found in the case of judgment for the occurrence of rejection while the dose of the immunosuppressive agent is reduced, it is judged that immunological tolerance is not acquired; and if no significant change in the expression level of the gene marker has been found while the dose of the immunosuppressive agent is reduced and withdrawal from the immunosuppressive agent is achieved, it is judged that immunological tolerance is acquired. For example, when the gene marker is a gene related substance related to a gene selected from the group consisting of Bc1211, cig5, Il18bp, andGbp2, if the expression level of the gene marker increases while the dose of an immunosuppressive agent is reduced, it is judged that immunological tolerance is not acquired; and if no significant change in the expression level of the gene marker has been found while the dose of the immunosuppressive agent is reduced and withdrawal from the immunosuppressive agent is achieved, it is judged that immunological tolerance is acquired.

As described above, by judging the presence or absence of immunological tolerance, it becomes possible to discontinue administration of an immunosuppressive agent which is intrinsically unnecessary, and thus to avoid the risk of infections associated with long-term administration of the immunosuppressive agent and/or side effects of the immunosuppressive agent. A vertebrate to be diagnosed may be a human or a vertebrate other than a human, such as a mouse, a rat, or the like.

In addition, the method for judging the presence or absence of immunological tolerance according to the present invention can also be used to develop tolerance-inducing methods or to screen for tolerance-inducing agents.

For example, a tissue or an organ is transplanted into a vertebrate, such as a mouse or a rat, and the animal is given a test substance, or the animal is treated by a test method and then its blood is drawn for monitoring the expression level of a gene marker. By performing the aforementioned procedure repeatedly by using multiple test substances or test methods, it is possible to screen for immunosuppressive agents and immunosuppressing methods as well as for tolerance-inducing agents and tolerance-inducing methods.

As an example of the tolerance-inducing agent, T cells can be desensitized by administration of partial peptides of a peptide which serves as an inducer of immunological tolerance, in which case it is possible to screen various partial peptides to select an effective partial peptide. In addition, the aforementioned procedure can be applied to the screening methods for a compound which selectively inhibits the ICOS/B7h pathway and/or the CD28/CTLA4:B7 pathway, which are supplementary signaling pathways. Further, by changing mode of use and/or mode of administration such that, for example, one or more kinds of immunosuppressive agents are used in a vertebrate into which a tissue or an organ has been transplanted, and monitoring the expression level of the gene marker in the blood drawn from the vertebrate, it is possible to develop an effective tolerance-inducing method.

It should be noted that examples of the aforementioned gene markers that can be used include a gene-related substance related to a gene selected from the group consisting of Bc1211, cig5, I118bp, and Gbp2. The vertebrate that can be used is exemplified by a vertebrate, such as a mouse, a rat, and the like.

### === Kit ===

The kit described herein is not limited to any specific one as long as it includes a reagent or apparatus for measuring the expression level of the gene marker. Examples of such reagents or apparatus include primers and reagents used for the quantitative RT-PCR methods, antibodies used for the Western blotting and/or the ELISA method. In addition, the kit may include a reagent for isolating RNA from blood, probes for hybridization for gene markers, anti-immunoglobulin secondary antibodies labeled with an enzyme, fluorescent substances, radioactive substances, etc., and a substrate for the enzyme. The enzymes that can be used include horseradish peroxidase (HRP), alkaline phosphatase, etc. , the fluorescent substances that can be used include Cy2, FluorX, Cy3, Cy3.5, Cy5, Cy5.5, Cy7, FITC (fluorescein isothiocyanate), rhodamine, etc., and the radioactive substance that can be used include ³H, ³²P, ³⁵S, ¹²¹I, ¹²⁵I, etc.

The aforementioned primers include a primer directed to one or more genes selected from the group consisting of Bc1211, cig5, I118bp, and Gbp2. The aforementioned antibodies that can be used include polyclonal and monoclonal antibodies which react specifically to a gene marker (polypeptides or proteins) related to a gene such as Bc1211, cig5, I118bp, and Gbp2. It should be noted that the kit according to the present invention may include one or more antibodies.

### EXAMPLES

Hereinafter, the present invention will be explained in more detail with reference to Examples and drawings.

### [Example 1] - Reference

By using Lewis rats as donors and isogenic (Lewis) rats as recipients, orthotopic liver transplantation was performed (non-rejection group; n = 3). In addition, by using BN rats as donors and allogeneic (Lewis) rats as recipients, orthotopic liver transplantation was performed. Three (immunosuppression group; n = 3) out of the six allogeneic recipients received an intramuscular injection of 3 mg/kg body weight (BW) of cyclosporine (manufactured by Novartis Pharmaceuticals Corporation; dissolved in saline) on the day of transplantation (day 0), followed by daily intramuscular injections of cyclosporine at the same dose. The remaining three rats did not receive any immunosuppressive agent (rej ection group; n=3). The Lewis and BN rats used were 8 to 10-week-old males and weighed between 200 and 300 g.

On post-operative day 7 (7POD) when rejection was pathologically diagnosed, laparotomy was performed on three rats in each of the non-rejection group, rejection group, and immunosuppression group; and peripheral blood (about 5 ml) was drawn from the inferior vena cava. Then, mRNA was extracted with the QIAamp RNA Blood Mini kit (manufactured by QIAGEN Inc.) and DNA was removed by DNase treatment. By using 20 µg of the mRNAs obtained from the peripheral blood, cDNAs labeled with Cy5 dye were obtained with the LabelStar Array kit (manufactured by QIAGEN Inc.) . These cDNAs were hybridized with Atlas Glass Rat 3.81 Microarray (manufactured by BD Biosciences Clontech) . The microarrays were scanned with an Axon GenePix 4000A scanner (manufactured by Axon Instruments) and the data were analyzed by using GenePix Pro3.0 Microarray analysis software (manufactured by Axon Instruments) (with global normalization). As a result, the genes (Bc1211 and Best5) whose expression levels had been markedly increased in the rejection group, compared with the non-rejection group or the immunosuppression group, were selected.

To precisely examine changes in the expression level of each of the genes selected here, their mRNAs were quantified. For Bc1211 and Best5, mRNA was quantified by using 25 ng of extracted mRNA together with respective primers and a probe (final concentrations: 900 nM each primer, 200 nM TaqMan probe; TaqMan Gene Expression Assays Rn00580568_g1 (Bc1211) or Rn00594081_m1 (Best5), Applied Biosystems), synthesizing cDNA with TaqMan Reverse Transcription Reagents (manufactured by Applied Biosystems) on an ABI PRISM 7900HT Detection System (manufactured by Applied Biosystems), and using rat β-actin as an internal control. For rat β-actin (NM031144), the TaqMan probe (final concentration: 100 nM) was (VIC)-CCATCACACCCTGGTGCC-(MGB); SEQ ID NO: 1); and the primers (final concentration: 200 nM each) were (5'-GCCGTCTTCCCCTCCAT (SEQ ID NO: 2) and 5'-AGGAGTCCTTCTGACCCATACC (SEQ ID NO: 3). In this quantitative mRNA analysis, post-transplantation expression levels of the gene markers were relatively quantified using the pre-transplantation expression levels of the gene markers as a standard (as "1") by the ΔΔCt method.

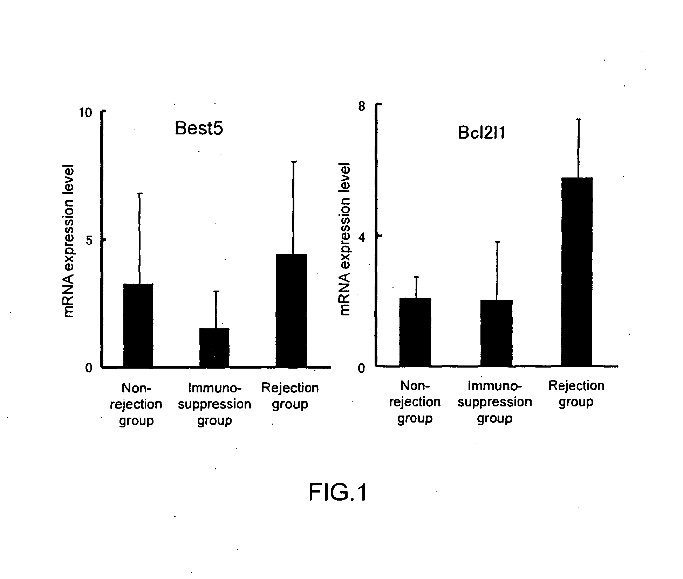

As a result, as shown in FIG. 1, it was revealed that, on 7POD when rejection was pathologically diagnosed, the expression levels of the mRNAs of Bc1211 and Best5 were increased due to rejection caused by orthotopic liver transplantation and suppressed by the administration of an immunosuppressive agent.

### [Example 2] - Reference

To examine whether the genes identified in Example 1 can serve as indicators of rejection or of the efficacy of an immunosuppressive agent, 200 µl of blood was drawn from the jugular vein of individual rats in the non-rejection group (n = 7), rejection group (n = 5), and immunosuppression group (n = 5) starting from the day before transplantation and daily afterward until post-operative day 6. On post-operative day 7, laparotomy was performed on individual rats, 200 µl of blood was drawn from the inferior vena cava, and expression levels of gene markers (Best5 andBc1211) were measured. The expression levels of the gene markers were measured by quantifying mRNAs, which had been extracted from peripheral blood in the same manner as described in Example 1.

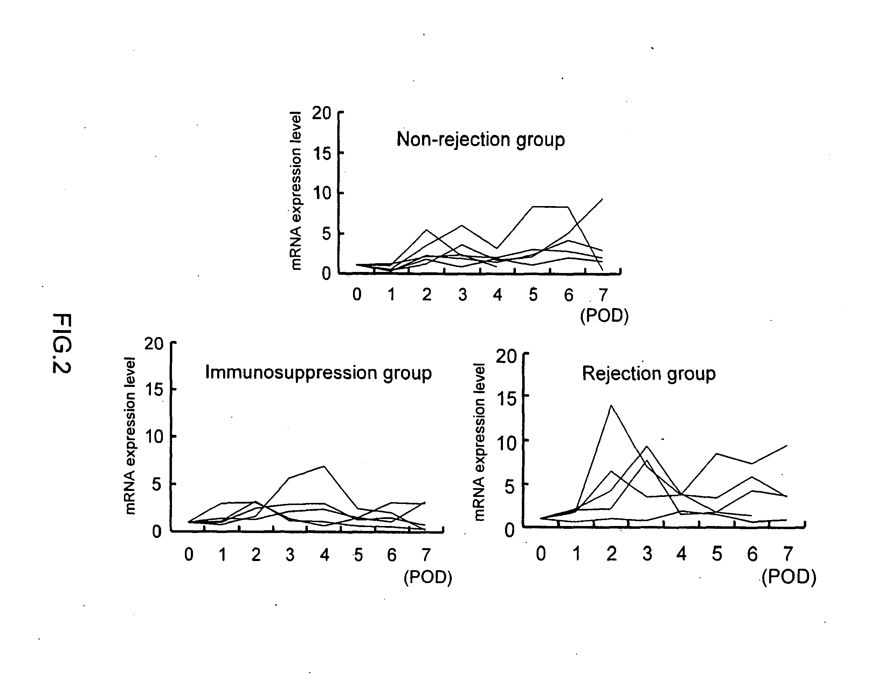

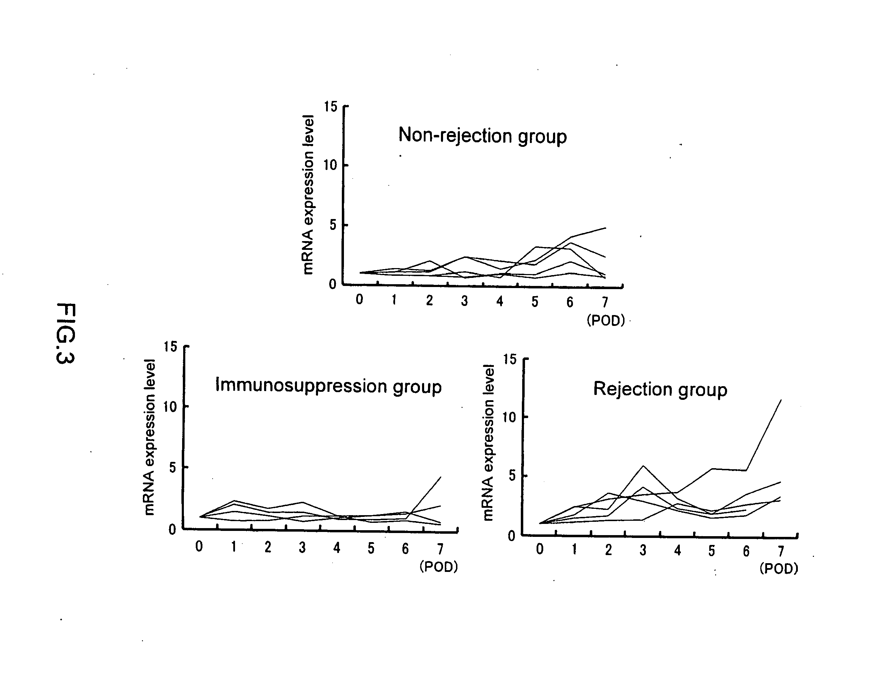

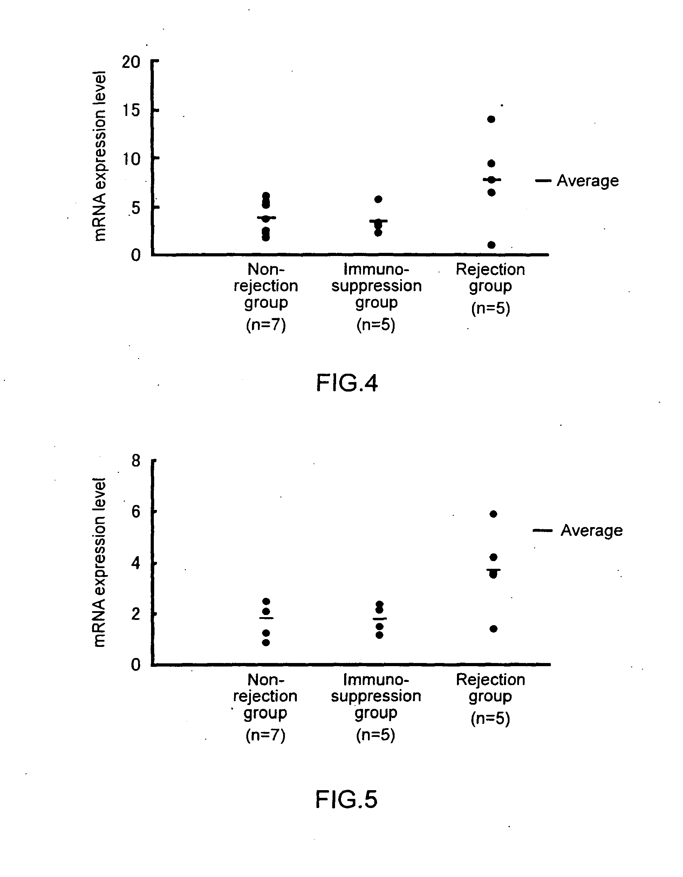

As shown in FIG. 2 (results of Best5), and FIG. 3 (results of Bc1211), it was revealed that in the immunosuppression group the expression levels of the gene markers were suppressed compared with the rejection group, to the level similar to or lower than that of the non-rejection group, whereas in the rejection group the expression levels of the gene markers Bc1211 and Best5 were increased at an earlier period (2POD to 3POD) before 7POD when rejection was diagnosed. Thus, on the assumption that Best5 and Bc1211 might serve as early markers of rejection, each gene's expression level on the day when the highest expression of the data till 3POD had been achieved was picked up and the average value for each group was obtained. As shown in FIG. 4 (results of Best5) and FIG. 5 (results of Bc1211), the expression levels of Best5 and Bc1211 were both increased by 3POD in the rejection group, indicating that these genes could serve as early markers of rejection.

### [Example 3] - Reference

By using Lewis rats as donors and isogenic (Lewis) rats as recipients, orthotopic liver transplantation was performed (non-rejection group; n = 3). In addition, by using ACI rats as donors and allogeneic (Lewis) rats as recipients, orthotopic liver transplantation was performed. Three (immunosuppression group; n = 3) out of the six allogeneic recipients received an intramuscular injection of 0.64 mg/kg BW of tacrolimus (manufactured by Fujisawa Pharmaceutical Co., Ltd.; dissolved in saline) on the day of transplantation (day 0), followed by daily intramuscular injections of tacrolimus at the same dose. The remaining three rats did not receive any immunosuppressive agent (rejection group; n=3) . The Lewis and ACI rats used were 8 to 10-week-old males and weighed between 200 and 300 g.

On post-operative day 5 (5POD) when a symptom of rejection was observed, peripheral blood (about 200 µl) was drawn from the jugular vein of three rats in each of the non-rej ection group, rejection group, and immunosuppression group. Then, mRNA was extracted with the QIAamp RNA Blood Mini kit (manufactured by QIAGEN Inc.) and DNA was removed by DNase treatment. 25 ng of the mRNAs obtained from the peripheral blood were hybridized following the protocol of Rat Expression Array 230A (Affymetrix), scanned and the data were analyzed (with global normalization) . As a result, the genes (Igifbp and Gbp2) whose expression levels had been markedly increased in the rejection group, compared with the non-rejection group or the immunosuppression group, were selected.

To examine whether the Igifbp gene and Gbp2 gene serve as indicators of rejection or of the efficacy of an immunosuppressive agent, 200 µl of peripheral blood was drawn from the jugular vein of individual rats in the non-rejection group (n = 6), rejection group (n = 5), and immunosuppression group (n = 3) starting from the day before transplantation and daily afterward until post-operative day 7, and the expression levels of these genes were measured. For measurement of the expression levels, mRNA was quantified by using 25 ng of mRNA extracted from the peripheral blood together with respective primers and a probe (final concentrations: 900 nM each primer, 200 nM TaqMan probe; TaqMan Gene Expression Assays Rn00584495_g1 (Igifbp) or Rn00592467_m1 (Gbp2), Applied Biosystems), synthesizing cDNA with TaqMan Reverse Transcription Reagents (manufactured by Applied Biosystems) on an ABI PRISM 7900HT Detection System (manufactured by Applied Biosystems), and using β-actin as an internal control, as described in Example 1. In this quantitative mRNA analysis, post-transplantation expression levels of the gene markers were relatively quantified using the pre-transplantation expression levels of the gene markers as the standard (as "1") by the ΔΔCt method.

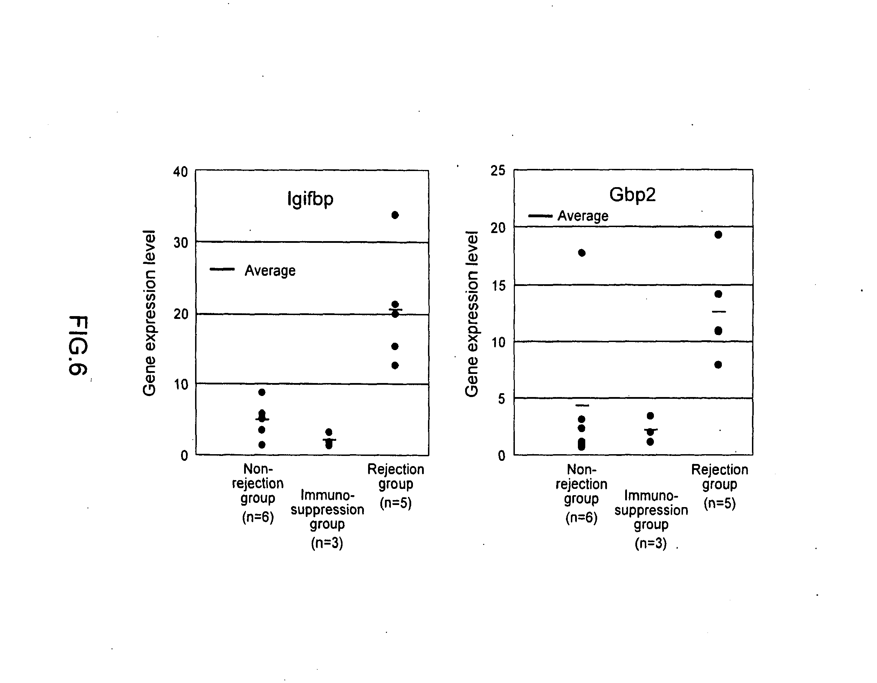

As a result, it was revealed that on 5POD, two days earlier than 7POD when rejection was pathologically diagnosed, the expression levels of the mRNAs of Igifbp and Gbp2 were increased due to rejection caused by orthotopic liver transplantation and suppressed by the administration of an immunosuppressive agent (Refer to FIG. 6).

### [Example 4]

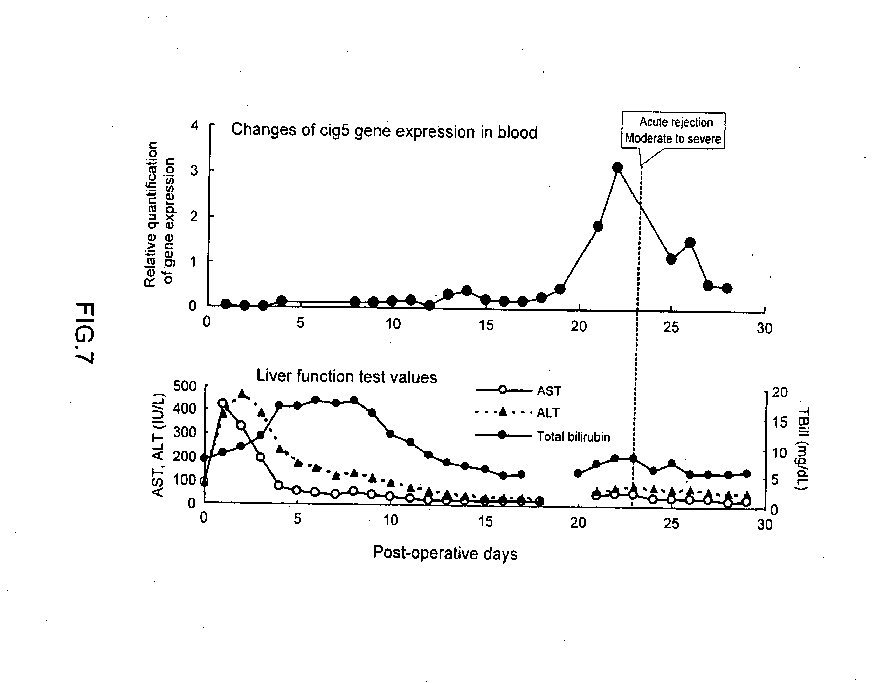

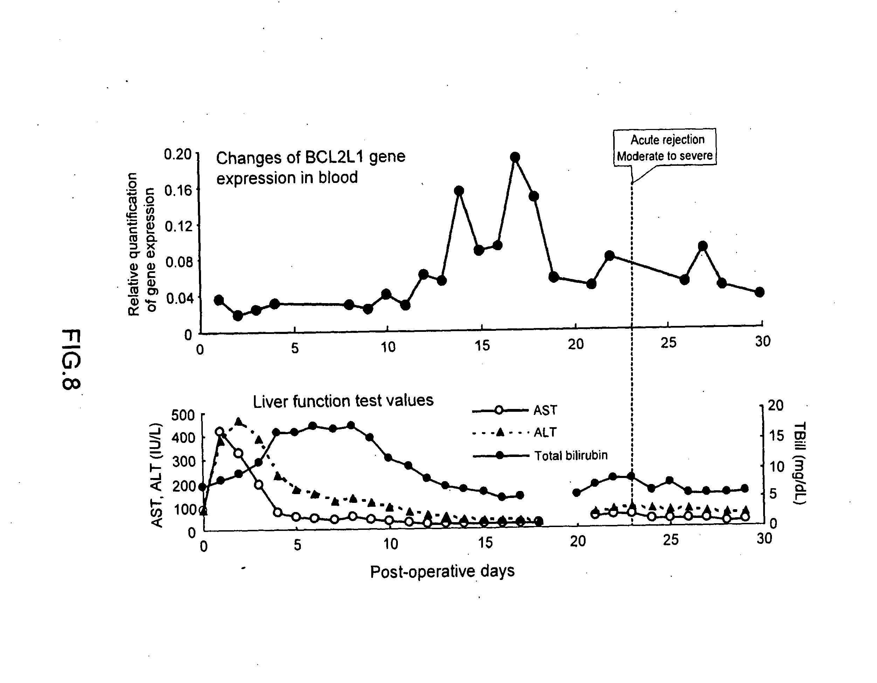

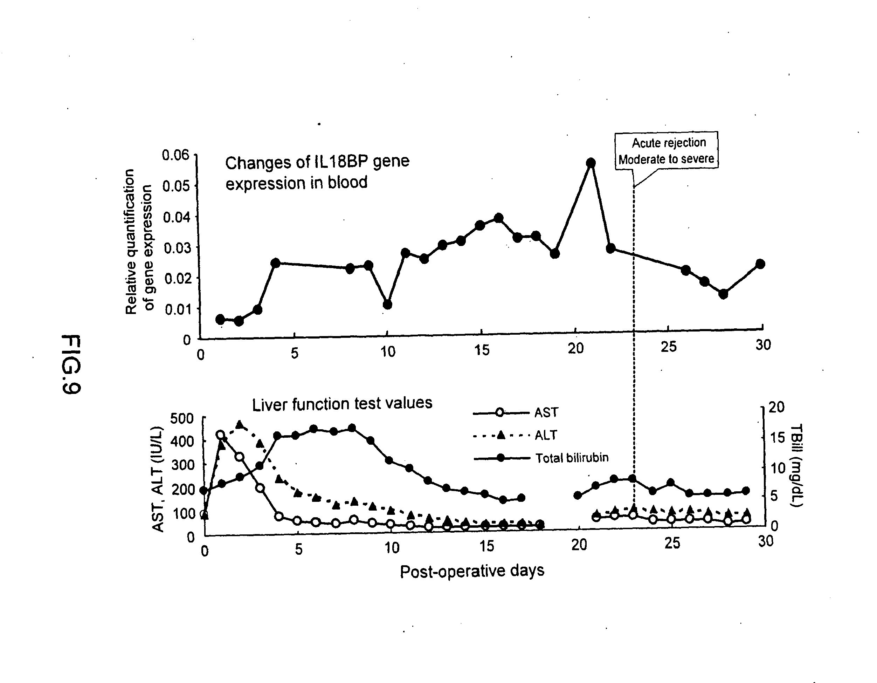

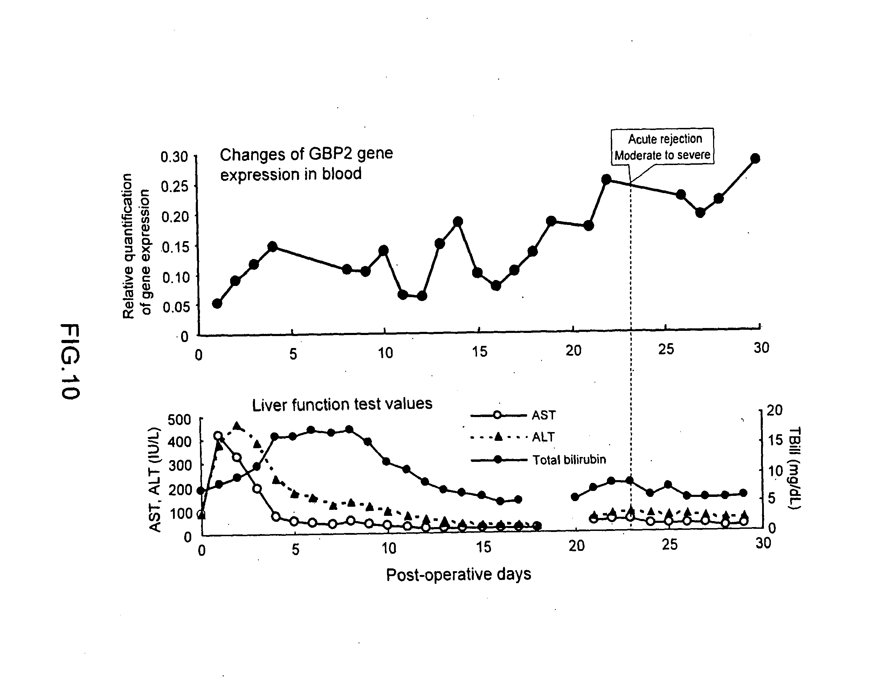

On the day shown in FIGs 7-10, before administration of cyclosporine, peripheral blood (about 200 µl) was drawn from the cubital vein of a patient (13-years old; male; 37 kg) with congenital biliary atresia who had undergone an ABO/Rh-compatible liver transplantation. Changes in the expression levels of each of the following genes in peripheral blood caused by the liver transplantation was examined: the cig5 gene (RSAD2 (NM 080657) : a homologous gene of the rat Best5 gene) ; BCL2L1 gene; IL18BP gene (a homologous gene of the rat Igifbp gene) ; and the GBP2 gene. The expression levels were measured by extracting mRNA from peripheral blood with the QIAamp RNA Blood Mini kit (manufactured by QIAGEN Inc.), removing DNA by DNase treatment, then quantifying mRNA using 25 ng of the extracted mRNA together with respective primers and a probe (TaqMan Gene Expression Assays; Applied Biosystems), synthesizing cDNA with TaqMan Reverse Transcription Reagents (manufactured by Applied Biosystems) on an ABI PRISM 7900HT Detection System (manufactured by Applied Biosystems), and using human β-action as an internal control.

For the cig5 gene, the primers used (final concentration: 900 nM each) were 5'-GGTGCCTGAATCTAACCAGAAGA (SEQ ID NO: 4) and 5'-ACTTGGAAGGGTCCTTCCGT (SEQ ID NO: 5) ; and the probe used (final concentration: 200 nM) was (FAM)- ATGAATATATGCGCTTTCT-(MGB) (SEQ ID NO: 6) . For the BCL2L gene, IL18BP gene, and GBP2 gene, the final concentrations of each of the primers and the probes were 900 nM and 200 nM, respectively (TaqMan Gene Expression Assays Hs00236329_m1 (BCL2L1), Hs00271720_m1 (IL18BP), and Hs00269759_m1 (GBP2), manufactured by Applied Biosystems). For human β-actin (NM001101), the TaqMan probe used (final concentration: 100 nM) was (FAM)-CAGGCACCAGGGCGTGATGGT-(TAMRA) (SEQ ID NO 7) and the primers used (final concentration: 200 nM each) were 5'-1GCCGCCAGCTCACCAT (SEQ ID NO:8) and 5'-AGGAATCCTTCTGACCCATGC (SEQ ID NO: 9). In this quantitative mRNA analysis, expression levels of the gene markers were relatively quantified using BD qPCR Human Reference cDNA (manufactured by BD Biosciences) as a control by the ΔΔCT method.

In addition, blood was drawn from the patient on the days shown in FIGs 7-10 and liver function tests (measurements of AST, ALT, and total bilirubin levels in blood) were also conducted. Rejection was diagnosed by liver biopsy.

As shown in FIGs 7-10, it was revealed that the expression levels of the cig5 gene, BCL2L1 gene, IL18BP gene, and GBP2 gene in peripheral blood were increased earlier than the time point when rejection was pathologically diagnosed by liver biopsy. It was further revealed that, on post-operative day 23, when rejection was pathologically observed, the expression levels of the cig5 gene, BCL2L1 gene, and IL18BP gene in peripheral blood were suppressed (refer to the expression level of each gene on and after post-operative day 25) by the steroid pulse therapy performed for the purpose of treating acute rejection. In contrast, the expression level of the GBP2 gene in peripheral blood was temporarily decreased by the same steroidpulse therapy. In addition, liver function tests showed that serum AST, ALT, and total bilirubin levels were nonspecifically increased irrespective of the time point when rejection is pathologically observed.

The above-described Examples suggested that the gene markers, Bc1211, cig5 (Best5), I118bp (Igifbp), Gbp2, etc., are useful as indicators for early diagnosis of rejection, judgment of the presence or absence of immunological tolerance, prediction of potential rejection or immunological tolerance, and/or the efficacy of an immunosuppressive agent.

### INDUSTRIAL APPLICABILITY

In accordance with the present invention, gene markers which enable diagnosis of rejection, evaluation of the efficacy of immunosuppressive agents, and judgment of the presence or absence of immunological tolerance; methods that can be quickly and conveniently performed by using the gene marker as an indicator for diagnosing rejection, evaluating the efficacy of immunosuppressive agents, identifying immunosuppressive agents, and judging the presence or absence of immunological tolerance; and methods for screening for immunosuppressive agents and immunological tolerance-inducing agents, can be provided.

## Claims

1. A gene marker for use as an indicator of rejection, an indicator of immunological tolerance, or as an indicator of the efficacy of an immunosuppressive agent,
the gene marker is a Cig5 gene, an mRNA as a transcript of the Cig5 gene, a peptide as a translation product of the Cig5 gene, or a protein as an end product of expression of the Cig5 gene.

2. The gene marker of claim 1, wherein the gene marker is for use as an indicator of rejection due to liver transplantation, as an indicator of immunological tolerance to liver transplantation, or as an indicator of the efficacy of an immunosuppressive agent on liver transplantation.

3. A method for diagnosing rejection,
comprising the step of measuring a change in the expression level of a gene marker according to claim 1 in blood obtained from a vertebrate.

4. The method for diagnosing rejection of claim 3, wherein the method is for diagnosing rejection due to liver transplantation.

5. A method for judging the presence or absence of immunological tolerance,
comprising the step of measuring a change in the expression level of a gene marker according to claim 1 in blood drawn from a vertebrate into which a tissue or an organ has been transplanted.

6. The method for judging the presence or absence of immunological tolerance of claim 5, wherein the tissue or the organ is a liver.

7. A method for judging the presence or absence of immunological tolerance according to claim 5 or 6, comprising the steps of:
monitoring the expression level of a gene marker in blood obtained from a vertebrate into which a tissue or an organ has been transplanted while reducing the dose of an immunosuppressive agent administered to the vertebrate after the transplantation; and
judging that the immunological tolerance is acquired in the case that no significant change in the expression level is found while the dose is reduced and withdrawal from the immunosuppressive agent is achieved.

8. A method for evaluating the efficacy of an immunosuppressive agent,
comprising the step of measuring a change in the expression level of a gene marker according to claim 1 in blood associated with a use of the immunosuppressive agent.

9. The method for evaluating the efficacy of an immunosuppressive agent of claim 8, wherein the method is for evaluating the efficacy of an immunosuppressive agent on liver transplantation.

10. A method for identifying an effective immunosuppressive agent for a vertebrate requiring administration of an immunosuppressive agent, comprising the steps of:
measuring the expression level of a gene marker according to claim 1 in the blood obtained from a plurality of individuals of the vertebrate family before and after having administered an equal amount of different immunosuppressive agents;
comparing the expression levels of the gene marker in the blood obtained from the individual between before and after the immunosuppressive agent has been administered; and
identifying the immunosuppressive agent having decreased the expression level of the gene marker most markedly by the
administration of the immunosuppressive agent.

11. The method for identifying an effective immunosuppressive agent of claim 10, wherein the immunosuppressive agent is for liver transplantation.

12. A method for screening for an immunosuppressive agent or an immunological tolerance-inducing agent, wherein:
a test substance has been administered to a vertebrate in which a tissue or an organ transplant causes immunological rejection;
monitoring the expression level of a gene marker according to claim 1 in the blood obtained from the vertebrate.

13. The method for screening of claim 12, wherein the tissue or the organ is a liver.

## Patentansprüche

1. Genmarker zur Verwendung als ein Indikator für die Abstoßung, ein Indikator für die immunologische Toleranz oder als ein Indikator für die Wirksamkeit eines Immunsuppressivums,
wobei der Genmarker ein Cig5-Gen, eine mRNA als ein Transkript des Cig5-Gens, ein Peptid als ein Translationsprodukt des Cig5-Gens oder ein Protein als ein Endprodukt der Expression des Cig5-Gens ist.

2. Genmarker nach Anspruch 1, wobei der Genmarker zur Verwendung als ein Indikator für die Abstoßung infolge einer Lebertransplantation, als ein Indikator für die immunologische Toleranz gegenüber einem Lebertransplantat oder als ein Indikator für die Wirksamkeit eines Immunsuppressivums auf das Lebertransplantat vorgesehen ist.

3. Verfahren zum Diagnostizieren einer Abstoßung, umfassend den Schritt des Messens einer Veränderung des Expressionsspiegels eines Genmarkers nach Anspruch 1 in von einem Vertebraten gewonnenem Blut.

4. Verfahren zum Diagnostizieren einer Abstoßung nach Anspruch 3, wobei das Verfahren zum Diagnostizieren einer Abstoßung infolge einer Lebertransplantation vorgesehen ist.

5. Verfahren zur Beurteilung der An- oder Abwesenheit einer immunologischen Toleranz,
umfassend den Schritt zum Messen einer Veränderung des Expressionsspiegels eines Genmarkers nach Anspruch 1 in von einem Vertebraten gewonnenem Blut, in den ein Gewebe oder ein Organ transplantiert wurde.

6. Verfahren zur Beurteilung der An- oder Abwesenheit einer immunologischen Toleranz nach Anspruch 5, wobei das Gewebe oder das Organ eine Leber ist.

7. Verfahren zur Beurteilung der An- oder Abwesenheit einer immunologischen Toleranz nach Anspruch 5 oder 6, umfassend die Schritte von:
Überwachung des Expressionsspiegels eines Genmarkers in von einem Vertebraten gewonnenem Blut, in den ein Gewebe oder ein Organ transplantiert wurde, während die Dosis eines an den Vertebraten nach der Transplantation verabreichten Immunsuppressivums reduziert wird; und
Beurteilung, dass die immunologische Toleranz in dem Fall erworben ist, wenn keine signifikante Veränderung des Expressionsspiegels gefunden wird, während die Dosis reduziert wird und das Absetzen des Immunsuppressivums erreicht wird.

8. Verfahren zur Bewertung der Wirksamkeit eines Immunsuppressivums,
umfassend den Schritt des Messens einer Veränderung des Expressionsspiegels eines Genmarkers nach Anspruch 1 im Blut, die mit der Anwendung des Immunsuppressivums assoziiert ist.

9. Verfahren zur Bewertung der Wirksamkeit eines Immunsuppressivums nach Anspruch 8, wobei das Verfahren zur Bewertung der Wirksamkeit eines Immunsuppressivums auf das Lebertransplantat vorgesehen ist.

10. Verfahren zur Identifikation eines wirksamen Immunsuppressivums für einen Vertebraten mit Bedarf an der Verabreichung eines Immunsuppressivums, umfassend die folgenden Schritte:
Messung des Expressionsspiegels eines Genmarkers nach Anspruch 1 in von einer Vielzahl von Individuen der Vertebratenfamilie gewonnenem Blut, bevor und nachdem eine gleiche Menge verschiedener Immunsuppressiva verabreicht wurde;
Vergleich der Expressionsspiegel des Genmarkers in von dem Individuum gewonnenem Blut, bevor und nachdem das Immunsuppressivum verabreicht wurde; und
Identifikation des Immunsuppressivums, welches den Expressionsspiegel des Genmarkers am ausgeprägtesten durch die Verabreichung des Immunsuppressivums vermindert hat.

11. Verfahren zur Identifikation eines wirksamen Immunsuppressivums nach Anspruch 10, wobei das Immunsuppressivum für eine Lebertransplantation vorgesehen ist.

12. Verfahren zum Screening eines Immunsuppressivums oder eines Mittels zur Induktion der immunologischen Toleranz, wobei:
eine Testsubstanz an einen Vertebraten verabreicht wird, in dem ein Gewebe- oder Organtransplantat eine immunologische Abstoßung hervorruft;
Überwachung des Expressionsspiegels eines Genmarkers nach Anspruch 1 in von dem Vertebraten gewonnenem Blut.

13. Verfahren zum Screening nach Anspruch 12, wobei das Gewebe oder das Organ eine Leber ist.

## Revendications

1. Marqueur génétique conçu pour être utilisé comme indicateur d'un rejet, comme indicateur d'une tolérance immunologique ou comme indicateur de l'efficacité d'un agent immunosuppresseur, où le marqueur génétique est le gène Cig5, un ARNm en tant que transcript du gène Cig5, un peptide en tant que produit de traduction du gène Cig5 ou une protéine en tant que produit final de l'expression du gène Cig5.

2. Marqueur génétique de la revendication 1, où le marqueur génétique est conçu pour être utilisé comme indicateur du rejet d'une transplantation hépatique, comme indicateur d'une tolérance immunologique à une transplantation hépatique ou comme indicateur de l'efficacité d'un agent immunosuppresseur sur une transplantation hépatique.

3. Méthode permettant de diagnostiquer un rejet, qui comprend l'étape consistant à mesurer un changement dans le niveau d'expression d'un marqueur génétique selon la revendication 1 dans le sang obtenu chez un vertébré.

4. Méthode de la revendication 3 permettant de diagnostiquer un rejet, où la méthode est conçue pour diagnostiquer le rejet d'une transplantation hépatique.

5. Méthode permettant de juger si une tolérance immunologique est présente ou absente, qui comprend l'étape consistant à mesurer un changement dans le niveau d'expression d'un marqueur génétique selon la revendication 1 dans le sang obtenu chez un vertébré chez qui un tissu ou un organe a été transplanté.

6. Méthode de la revendication 5 permettant de juger si une tolérance immunologique est présente ou absente, où le tissu ou l'organe est un foie.

7. Méthode de la revendication 5 ou 6 permettant de juger si une tolérance immunologique est présente ou absente, qui comprend les étapes consistant à :
surveiller le niveau d'expression d'un marqueur génétique dans le sang obtenu chez un vertébré chez qui un tissu ou un organe a été transplanté tout en abaissant la dose d'un agent immunosuppresseur administré au vertébré après la transplantation; et
juger qu'une tolérance immunologique est acquise si aucun changement significatif du niveau d'expression est détecté alors que la dose est abaissée et qu'un sevrage de l'agent immunosuppresseur est produit.

8. Méthode permettant d'évaluer l'efficacité d'un agent immunosuppresseur, qui comprend l'étape consistant à mesurer un changement, associé à l'utilisation de l'agent immunosuppresseur, dans le niveau d'expression d'un marqueur génétique selon la revendication 1 dans le sang.

9. Méthode de la revendication 8 permettant d'évaluer l'efficacité d'un agent immunosuppresseur, où la méthode est utilisée pour évaluer l'efficacité d'un agent immunosuppresseur sur une transplantation hépatique.

10. Méthode permettant d'identifier un agent immunosuppresseur efficace chez un vertébré chez qui l'administration d'un agent immunosuppresseur est requise, qui comprend les étapes consistant à :
mesurer le niveau d'expression d'un marqueur génétique selon la revendication 1 dans le sang obtenu chez une pluralité d'individus de la famille du vertébré avant et après l'administration d'une quantité égale de différents agents immunosuppresseurs ;
comparer les niveaux d'expression du marqueur génétique dans le sang obtenu chez l'individu avant et après l'administration de l'agent immunosuppresseur ; et
identifier l'agent immunosuppresseur dont l'administration est associée à la diminution la plus importante du niveau d'expression du marqueur génétique.

11. Méthode de la revendication 10 permettant d'identifier un agent immunosuppresseur efficace, où l'agent immunosuppresseur est utilisé pour une transplantation hépatique.

12. Méthode de dépistage d'un agent immunosuppresseur ou d'un agent induisant une tolérance immunologique, qui consiste à :
administrer la substance testée à un vertébré chez qui la transplantation d'un tissu ou d'un organe cause un rejet immunologique ;
surveiller le niveau d'expression d'un marqueur génétique selon la revendication 1 dans le sang obtenu chez le vertébré.

13. Méthode de dépistage de la revendication 12, où le tissu ou l'organe est un foie.
